# EUROPÄISCHE PATENTSCHRIFT

(11) **EP 4 387 570 B1**
(45) Veröffentlichungstag und Bekanntmachung des Hinweises auf die Patenterteilung: **01.10.2025**
(21) Anmeldenummer: 22768662.3
(22) Anmeldetag: 19.08.2022
(51) Int. Cl.: A61F 2/24

(54) **HERZKLAPPENPROTHESE**
HEART VALVE PROSTHESIS
PROTHÈSE VALVULAIRE CARDIAQUE

(30) Priorität: 19.08.2021 DE 102021121556
(43) Veröffentlichungstag der Anmeldung: 26.06.2024
(73) Patentinhaber: MITRASTREAM HEART VALVE TECHNOLOGY GMBH, 76676 Graben-Neudorf (DE)
(72) Erfinder: JUNG, Johannes, 74889 Sinsheim (DE)
(74) Vertreter: Fritzsche, Thomas
(86) Internationale Anmeldenummer: PCT/EP2022/073232
(87) Internationale Veröffentlichungsnummer: WO 2023/021199

(56) Entgegenhaltungen:
- US-A1- 2009 138 079
- US-A1- 2015 025 622
- US-A1- 2016 206 424
- US-A1- 2017 065 409
- US-A1- 2017 367 822
- US-A1- 2019 000 614
- US-A1- 2019 046 315
- US-A1- 2020 222 180

## Beschreibung

Die Erfindung betrifft eine Herzklappenprothese, die an einem Haltegerüst befestigt ist, insbesonders eine Mitralklappenprothese, die minimalinvasiv mittels eines Katheters transvenös in die Durchtrittsöffnung zwischen Vor- und Hauptkammer implantierbar ist.

Mit zunehmendem Alter nimmt die Häufigkeit erworbener Herzklappenfehler zu. Die Aortenklappenstenose wird mittlerweile überwiegend mittels kathetergestütztem Klappenersatz (TAVI) behandelt. Die Verankerung der TAVI Klappen ist aufgrund der meist vorhandenen Kalkablagerungen der nativen Aortenklappe gut möglich.

Bei den erworbenen Erkrankungen der Mitralklappe spielen Undichtigkeiten (Insuffizienzen) die Hauptrolle. In der Regel ist die Verkalkung bei der Mitralklappe jedoch nicht ausreichend, um eine künstliche Herzklappe daran verankern zu können. Weiterhin ist die Mitralklappe, welche den Blutstrom zwischen linker Herzvorkammer und linker Herzhauptkammer kontrolliert, aufgrund ihrer Lage minimalinvasiv nur schwer zugänglich, da hier enge Biegungen bzw. Kurven überwunden werden müssen, was eine katheterbasierte Implantation sehr erschwert. Weiterhin ist die Funktion der Mitralklappe deutlich komplexer als die der Aortenklappe.

Eine klinisch bedeutsame Undichtigkeit der Mitralklappe (Mitralklappeninsuffizienz) betrifft in den westlichen Industrienationen ca. 8% der Bevölkerung über 75 Jahre und geht ähnlich wie die Aortenklappenstenose mit einer deutlich erhöhten Sterblichkeit einher (Nkomo VT, Gardin JM, Skelton TN, Gottdiener JS, Scott CG, Enriquez-Sarano M. Burden of valvular heart diseases: a population-based study. Lancet 2006;368:1005-11). Dennoch werden lediglich 2% der Patienten einer chirurgischen Therapie unterzogen (Head SJ, van Leeuwen WJ, Van Mieghem NM, Kappetein AP. Surgical or transcatheter mitral valve intervention: complex disease requires complex decisions. EuroIntervention 2014;9:1133-5). Das hohe chirurgische Operationsrisiko und das Fehlen eines einfach und sicher durchführbaren katheterbasierten Klappenersatzes dürften die Gründe für die unzureichende Behandlung sein.

Derzeit kommen meist reparative Verfahren zum Einsatz wie der MitraClip. Eine schwierige Lernkurve sowie mangelnde Wirksamkeit gehören zu den Hauptlimitationen. Seit 2012 befinden sich per Katheter implantierbare Mitralklappen in der Entwicklung. Die meisten Systeme versuchen die bei der Aortenklappe erfolgreiche Technologie (TAVI) auf die Mitralklappe zu übertragen, obwohl beide Klappen einen vollkommen unterschiedlichen Aufbau und Funktionsweise haben. Weitere Nachteile sind unter anderem, dass die Systeme entweder einen transapikalen Zugangsweg erfordern (offene chirurgische Operation), zunächst die Einlage einer Verankerungsstruktur im Bereich des Mitralklappenringes erfordern, zu einer Verlegung des linksventrikulären Ausflusstraktes führen, zu dicke (für die Aortenklappe optimierte) Segel haben oder aufgrund nicht physiologischer Bauformen die natürliche Anatomie verändern. All diese Faktoren führen zu erhöhten Komplikationen bei der Implantation und selbst bei technischem Erfolg dennoch zu einem erhöhten thrombotischen Risiko (Head SJ, van Leeuwen WJ, Van Mieghem NM, Kappetein AP. Surgical or transcatheter mitral valve intervention: complex disease requires complex decisions. EuroIntervention 2014;9:1133-5.). Ein ,ideales' System sollte mittels alleinigem transvenösem / transseptalem Zugangsweg implantierbar sein, eine schlanke Bauform aufweisen, die sich in die natürlichen anatomischen Gegebenheiten des Mitralklappenringes und Halteapparates einfügt, und eine schlank aufbauende möglichst bikuspide Klappe mit einem Flussprofil analog zur natürlichen Mitralklappe aufweisen. Dabei sollte möglichst keine Beeinträchtigung des linksventrikulären Ausflusstraktes stattfinden, was einen Nachteil der meisten derzeit vorgeschlagenen Konzepte darstellt.

In der Literatur sind runde, ovale oder D-artige Querschnitte beschrieben. Keiner dieser Querschnitte erlaubt es, den Stent der natürlichen Form der Durchtrittsöffnung der Mitralklappe exakt anzupassen. So beschreibt beispielsweise die WO 2013/075215 eine mittels Katheter implantierbare Mitralklappe Prothese die aus einem D-förmigen stentartigen Trägering mit daran angebrachten Flügelklappen besteht. Der Trägering weist einen D-förmigen Querschnitt auf. Hierdurch kommt es zu einer unphysiologischen Änderung des natürlichen Klappenapparates.

Aus der US 2016/0331527 A1 ist eine Herklappenprothese bekannt, bei der die Herklappen an einer drahtförmigen Röhre mit einem inneren- und äußeren Wand befestigt sind.

Der Querschnitt der röhrenförmigen Drahtform, kann dabei sowohl Rund- als auch Nierenförmig oder auch in Form eines großen D ausgestalltet sein.

In der EP 3 456 293 A1 ist ein Implantiersystem für eine Herzklappenprothese beschrieben. Dabei sind die Herzklappen an ein expandierbares gitterförmiges Ankersystem befestigt, dass zur Vorhofseite einen kragenförmigen erweiterten Radius aufweist.

Die US 2017/065409 A1 offenbart eine Herzklappenprothese zur minimalinvasiven intravenösen Implantation.

Die Erfindung hat daher zum Ziel, eine leicht transvenös verabreichbare oder applizierbare Herzklappe, insbesonders eine Mitralklappe bereitzustellen, die einfach und problemlos implantierbar ist. Darüber hinaus hat die Erfindung zum Ziel, eine vorhandene durch Überdehnung der Klappenfäden hervorgerufene Ventrikeldilatation auf einfache Weise zu verbessern.

Des Weiteren soll zur Implantation der Herzklappenprothese vorzugsweise auch ein sehr schlankes und flexibles Implantationsbesteck bereitgestellt werden, mit dem diese einfach zu positionieren und freigesetzt werden kann, sich bereits bei Freisetzung vom Katheter in der richtigen anatomischen Ausrichtung befindet, sich zusätzlich selbst ausrichten kann. Die Herzklappenprothese soll vorzugsweise die Form und Funktion der natürlichen Mitralklappe imitieren und insbesonders die natürlichen Sehnenfäden derart modifizieren, dass ein negatives Remodelling des linken Ventrikels stattfindet. Sie soll auch vorzugsweise zu keiner Obstruktion des linksventrikulären Ausflusstraktes führen und vom Flussprofil mit der Anlage aus zwei Segeln dem natürlichen Flussprofil entsprechen, so dass es nicht zu thrombotischen Komplikationen kommt.

Diese Ziele werden durch die in den Ansprüchen definierten Merkmale erreicht.

Erfindungsgemäß wird vorgeschlagen, einen Herzklappenflügel oder ein Herzklappensystem an eine stentartige Halterung, im folgenden auch Stent genannt, mit einer optimal an die Herzanatomie angepassten Form zu befestigen, welches über hierfür optimierte Implantationskatheter an den Ort der Herzklappe eingeführt und dort abgelegt werden kann. Erfindungsgemäß hat es sich als vorteilhaft erwiesen, wenn der zylinderförmige Stent nicht völlig kreisförmig rund ausgebildet ist, sondern einen hiervon in Fließrichtung abweichenden Querschnitt aufweist, der die Form einer Niere, Bohne oder auch Banane hat. Der Querschnitt weist eine maximale Länge a, eine maximale Breite b und eine minimale Breite c auf. Dabei ist a größer als b und b ist größer als **c.** Die Größe a entspricht dem Durchmesser eines Kreises der die Herzklappe umrandet.

Die Nierenform, insbesondere die äußere Rundung, lässt sich zweckmäßigerweise durch einen um den Endpunkt der kleinen Breite c kreisähnlichen Umfang beschreiben. Im einfachsten Fall stellt die Rundung ein Kreis mit einem Mittelpunkt dar, der im Inneren der Nierenform am Ende der mittleren kleinen Breite c liegt, d. h. der in der Tiefe der nierenförmigen Einbuchtung. Dieser Mittelpunkt kann bezüglich der Breite a um +/- 20%, insbesondere um +/- 10%, wobei +/- 5% bevorzugt ist, schwanken. Dadurch entsteht ggf. ein ovalförmiger oder ein ellipsenförmiger Querschnitt. Ist dies der Fall, dann wird die Länge a ungleich geteilt und die beiden Teile sind nicht mehr gleich lang.

Der erfindungsgemäß, bevorzugte nierenförmige Querschnitt weist die maximale Länge a, typischerweise 20 - 45 mm, insbesondere 25- 40 mm und vorzugsweise 28 - 38 mm auf, die große Breite b beträgt typischerweise 10- 30 mm, wobei 13 - 28 mm bis 15 - 25 mm bevorzugt sind. Die mittig liegende kleine Breite c beträgt typischerweise 5 - 25 mm, wobei 7 - 23 mmm bzw. 9 - 20 mm bevorzugt sind. Dabei ist a größer als b und b stets größer als c.

Erfindungsgemäss bevorzugt ist ein Querschnitt, der die Form zweier gegeneinander liegender D-Buchstaben aufweist, bei der ein D seitenverkehrt angeordnet ist, wobei die geraden senkrechten Striche der beiden D aneinander liegen und der Querschnittsrand durch unterschiedliche Wölbungen bzw. Krümmungen des D gebildet wird. Bei dieser Form entfällt die bei den zuvor beschriebenen Querschnittsformen definierte Länge c, und wird durch die Summe d der jeweils maximalen Abstände (maximale Bauchwölbung) der beiden gegeneinander liegenden D-Buchstaben ersetzt, der aus der Summe des großen Abstandes (maximale Bauchwölbung) d₁ und des kleinen Abstandes (maximale Wölbung) d₂ besteht jeweils gemessen von der gedachten geraden senkrechten Linie des D. Dabei ist a größer d. Dieser Abstand d₁ (große Wölbung, bzw. großer Bogen) entspricht dann der Länge b der zuvor beschriebenen Querschnittsformen. Am Berührungspunkt der gebogenen Linie der beiden D, gehen die Kurven ohne einen Knick ineinander über. In einer besonderen Ausführungsform kann dieser Bereich für eine geringe Länge sogar kurz gerade ausgebildet sein.

Für die Querschnittslängen a und d₁ gelten die zuvor beschriebenen Maße. Für die geringere Aufwölbung des kleineren D beträgt der maximale Abstand d₂ von der gedachten Aufstrich- bzw. der geraden Linie des Buchstabens D mindestens 0,5 mm, vorzugsweise mindestens 0,75 mm, wobei mindestens 1 mm, insbesonders mindestens 2,5 mm bzw. 3,0 mm besonders bevorzugt ist. In einer zweckmäßigen Ausführungsform beträgt die Aufwölbung mindestens 7,5 mm. Das Verhältnis der beiden Höhen von der gedachten geraden d-Linie beträgt zwischen dem großen d₁ (kleinerer Radius) zum kleinen d₂ (größerer Radius) also d₁ zu d₂ höchstens 20:1, insbesonders mindestens 2:1, wobei Verhältnisse von höchstens 5:1 höchstens bzw. höchstens 10:1 und insbesonders höchstens 15:1 bevorzugt sind. Die Höhe des jeweiligen der gedachten D-Rückenlinie a beträgt je nach der gewünschten Herzklappengröße 15 mm bis 50 mm, insbesonders 20 bis 45 mm.

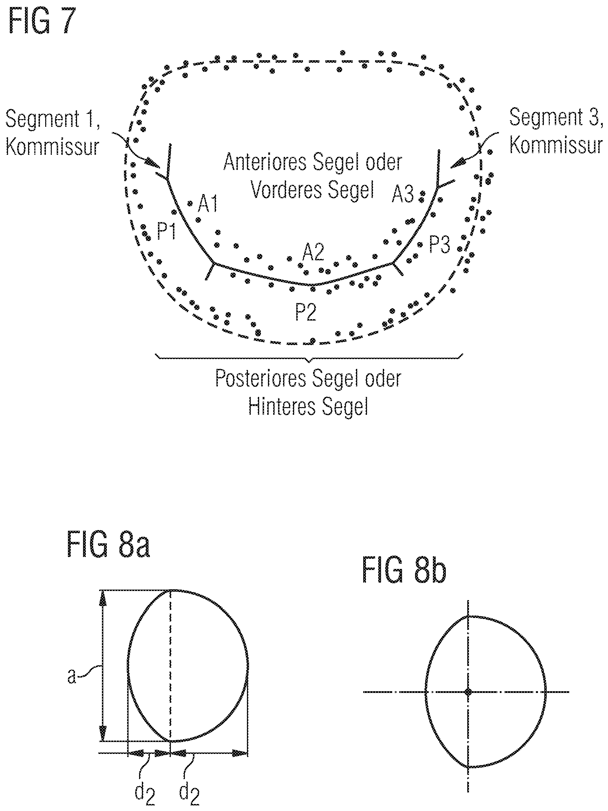

In einer weiteren bevorzugten Ausführungsform ist der Stent auf der Seite der kleineren Wölbung mit dem Abstand d₂(großer Radius) eine geringere Stegbreite auf als auf der Seite der größeren Wölbung (mit dem Abstand d₁). Dabei beträgt die Stegseite auf der größeren Wölbung typischerweise 0,3 mm bis 0,6 mm, vorzugsweise mindestens jedoch 0,35 mm und höchstens 0,4 mm bzw. 0,55 mm. Diese Stegseite wird auf der Seite der flacheren Wölbung (großer Radius) dünner ausgestaltet und beträgt höchstens 55% der Stegbreite auf der Seite mit der größeren Wölbung. Die minimalste Stegbreite beträgt in einer bevorzugten Ausführungsform 10%, insbesonders 20 bzw. 25% der Stegbreite auf der stärker gewölbten Seite (in Fig. 8 rechts dargestellt).

Mit der erfindungsgemäßen Prothese, insbesonders durch die unterschiedlich ausgestaltete Dicke der Stentstege ist es möglich, dass sich die eingesetzte Herzklappenprothese, insbesonders in ihrem Stentbereich, dem schlagenden Herz anpasst. Es hat sich nämlich gezeigt, dass die Ausführungsform mit der dünneren bzw. schmaleren Stegbreite der bewegliche Herzmuskel die Stentprothese beim Kontrahieren mitbewegen kann und nicht durch den starken Gegendruck des Stentes relativ starr bleibt. Damit ist es möglich, dass sich der Stent der natürlichen Herzmuskelbewegung anpasst bzw. die Durchtrittsöffnung auch in ihrer beweglichen Form sicher abdichtet.

Vorzugsweise nimmt die Fläche des Querschnitts des stentartigen Gitters in Fließrichtung von der Vorkammer kommend erst ab und nach der Durchtrittsöffnung zur Hauptkammer wieder zu, sodass eine ggf. geringfügige taillenförmige Einschnürung entsteht, die von der Herzmuskulatur umfasst wird. Auf diese Weise sitzt das stentartige Gitter oder Gerüst der Prothese fest in der Durchtrittsöffnung des Herzens.

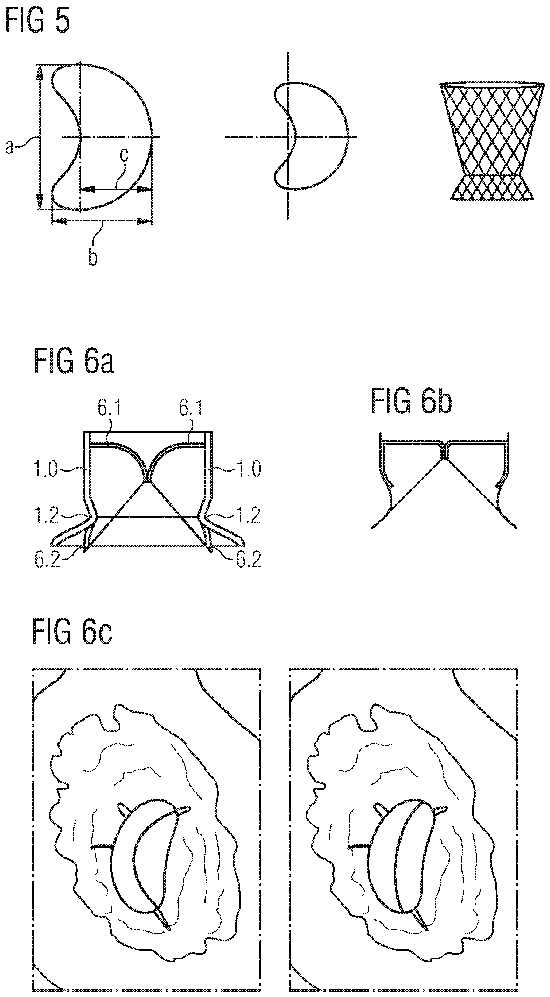

Der wie in Fig. 6 dargestellte Querschnitt ist vorzugsweise ein an der Vorhofseite eingeschnürter Zylinder. Er wird durch zwei verschiedene Kegelstümpfe dargestellt. Beim obereren Kegelstumpf beträgt die Höhe zwischen 51 bis 80%, beim unteren Kegelstumpf 49 bis 20% bezogen auf die Gesamtlänge des Stents.

Die erfindungsgemäße stentartige Klappenflügelhalterung weist eine, sich an die natürliche Form der Mitralklappe anpassende Form auf, die von einer kreisrunden Form deutlich abweicht und im Querschnitt eine Bohnenform, Nierenform oder Bananenform ausbildet, die in allen Querschnittsebenen (bezogen auf die Fließrichtung) symmetrisch oder unsymmetrisch sein kann.

In einer bevorzugten Ausführungsform hat der Stent im Bereich der Durchtrittsöffnung zwischen Vor- und Hauptkammer eine größere Dimension bzw. Fläche als die natürliche Umgebung, wodurch gegebenenfalls dilatierte Klappenfäden wieder gespannt werden.

In einer weiteren bevorzugten Ausführungsform weist das stentartige Gitter oder die Haltevorrichtung auf der zur Vorkammer liegenden Seite und/oder auf der zur Herzkammer liegenden Seite kleine, nach außen gerichtete Halteelemente oder Widerhaken auf. Auf diese Weise ist es möglich das stentartige Halteelement fest an der Klappenöffnung zu verankern.

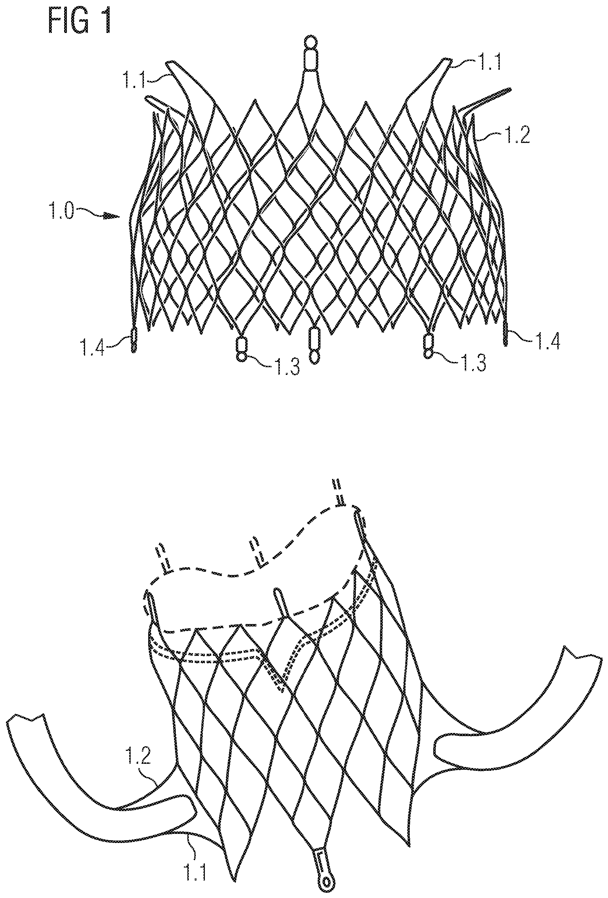

Fig. 1 zeigt einen Stent mit umlaufenden atraumatischen Halteelementen. Die Zahl der Halteelemente liegt dabei zwischen 2 und 15, typischerweise mindestens vier, wobei mindestens 8 bevorzugt sind.

An das Halteelement bzw. die stentartige Haltevorrichtung werden die Klappensegel befestigt.

Im Bereich des natürlichen posterioren Segels zeigt der Stent bzw. die Herzklappenhalterung bzw. -gerüst einen gebogenen Verlauf mit unterschiedlichen Kurvenradien auf (PML; P1 Abschnitt zur vorderen Kommissur, P2 mittlerer Abschnitt des PML und P3 Segment zur hinteren Kommissur) (Figur 7), wobei sich die Kurvenradien voneinander unterscheiden können, insbesondere besteht keine vollständige Spiegelsymmetrie zwischen P1 und P3 Segment. Dabei weist die Krümmung im Bereich des P2 Segments den größten Radius auf, P1 und P3 haben einen kleineren Radius, wobei erfindungsgemäß, der Radius von P1 kleiner als P3 ist oder von P1 größer als P3 ist, möglich sind auch identische Kurvenradien im Bereich von P1 und P3.

In einer weiteren bevorzugten Ausführungsform ist die Form der Herzklappenhalterung im Bereich des anterioren Segels (AML) an die natürliche Anatomie der Mitralklappe angepasst.

Bezogen auf die gedachte, zum nativen PML hin gebogene Kurve, die sich zwischen den Kommissuren der natürlichen Mitralklappe erstreckt, ist die Form des Stents bzw. der Herzklappenhalterung bzw. -gerüstes in den Kommissur-nahen Bereichen des A1 und A3 Segmentes eine nach außen Richtung LVOT geführte Kurve, die dann im mittleren Abschnitt des A1 und A3 Segmentes oder im jeweiligen Übergang zum A2 Segment die gedachte Kurve zwischen den Kommissuren nach innen Richtung natives PML kreuzt und diese in einer bogenförmigen Kurvenform weiterführt. Dabei sind die jeweiligen Kurvenradien typischerweise nicht symmetrisch zwischen A1 und A3 Segment und die Kurvenform innerhalb des A2 Segmentes ebenfalls nicht spiegelsymmetrisch. Die jeweiligen Radien können gleich, größer oder auch kleiner sein.

Die physiologische Funktion der Mitralklappe beinhaltet zusätzlich eine Bewegung in der dritten Dimension in Abhängigkeit vom Herzzyklus. Hierzu erlaubt die hier vorgestellte Mitralklappe eine Verformbarkeit während des Herzzyklus sowohl auf Höhe des Klappenrings als auch in Richtung des linken Vorhofs und des linken Ventrikels. Durch die Anpassung an die natürliche Anatomie der nativen Mitralklappe wird zusätzlich ein besserer Sitz und besserer Halt der Prothese ermöglicht. Darüber hinaus hat sich gezeigt, dass diese Form selbstzentrierend wirkt.

Mit der erfindungsgemäßen Prothese bleibt der natürliche Halteapparat der Klappe intakt, da die Prothese in der natürlichen Klappenöffnung befestigt wird, und zwar ohne die sonstigen anatomischen Gegebenheiten wesentlich zu ändern. Darüber hinaus hat es sich als besonders zweckmäßig erwiesen, den Stent, an dem die eigentliche Klappensegel befestigt sind, größer zu wählen, als es dem bisherigen Durchmesser der Durchtrittsöffnung entspricht. Auf diese Weise ist es möglich, dilatierte oder überdehnte Klappenfäden, welche die eigentlichen Klappen halten, wieder zu spannen.

Dadurch kommt es zusätzlich zu einer erhöhten Vorspannung der natürlichen Sehnenfäden mit ihren zugehörigen Papillarmuskeln, so dass im günstigen Falle ein negatives Remodelling des linken Ventrikels stattfindet. Dies wird dadurch begünstigt, dass die erfindungsgemäße Form und Funktion der Klappenprothese die natürliche Mitralklappe imitiert und somit zu einer in allen Ebenen gleichmäßigen verbesserten Vorspannung der natürlichen Sehnenfäden führt.

Durch die an die natürliche Anatomie angepasste Positionierung wird in allen Fällen auch bei einer hierfür ungünstigen Anatomie die Obstruktion des linksventrikulären Ausflusstraktes verhindert.

Die Klappensegel sind typischerweise bikuspid angelegt, es können aber auch Klappenprothesen mit ggf. 3 oder sogar 4 Einzelsegel zur Anwendung kommen.

In das Klappengerüst integrierte und darin befestigte Haltefäden imitieren die natürlichen Sehnenfäden und verhindern ein Durchschlagen der Segel in den Vorhof bei der Kontraktion des Ventrikels. Die Zahl der Haltfäden pro Segel liegt dabei zwischen 1 und 15, typischerweise mindestens drei, wobei mindestens vier Bevorzugt sind. Die maximale Anzahl der Haltefäden beträgt 15 wobei maximal 9 bzw. 7 bevorzugt sind.

Die Klappensegel bilden in ihrer Kontaktfläche eine Adaptionslinie aus, entlang derer die Klappensegel aneinander anliegen und den Rückfluss verhindern/verschließen. Die Adaptationslinie der Segel orientiert sich typischerweise an der Form des Stents, kann davon jedoch auch abweichen. Typischerweise läuft die Adaptationslinie von dem Ort der natürlichen Kommissur zwischen A1/P1 zu dem Ort der natürlichen Kommissur zwischen A3/P3, kann jedoch auch bis zu jeweils 5 mm, insbesondere 4 mm bzw. 3 mm in Richtung anterior oder posterior verschoben sein. Die Krümmung der Adaptationslinie folgt der Adaptationslinie der natürlichen Klappe, kann hiervon jedoch auch um bis zu jeweils 5 mm, insbesondere 4 mm bzw. 3 mm in Richtung anterior oder posterior verschoben sein. In einer bevorzugten Ausführung werden zur seitlichen Abdichtung die Segel vorhofseitig an der Innenseite des Stents in Richtung Ventrikel fortgeführt, wie dies beispielweise in Figur 6 b dargestellt ist.

Zum Einbringen eines solchen Herzklappensystems ist es zunächst erforderlich, dies in ein Applikationsbesteck/Kathetersystem zu laden, welches transvenös eingeführt wird, und zwar entweder über eine anatomisch caudal gelegene Vene (typischerweise perkutan die Vena femoralis oder chirurgisch dargestellt im Becken gelegene Venen, aber darauf nicht beschränkt) oder eine cranial gelegene Vene (typischerweise perkutan oder chirurgisch eine Jugularvene oder die Vena subclavia oder axillaris, aber darauf nicht beschränkt). Das Applikationsbesteck besitzt die notwendigen Abmessungen und vor allem Flexibilität, den Weg zur Mitralklappe per transseptalem Zugangsweg vom rechten in den linken Vorhof und weiter Richtung linker Ventrikel durch die natürliche Mitralklappe über einen zuvor gelegten Führungsdraht zu folgen auch wenn der Abstand zwischen transseptaler Punktionshöhe und Mitralklappenebene typischerweise nicht mehr als 4 cm, typischerweise nicht mehr als 3 cm, vorzugsweise nicht mehr als 2 cm beträgt.

Zum Positionieren der erfindungsgemäßen Prothese wird zweckmäßigerweise ein sogenannter steuerbarer Katheter verwendet. Bei einem solchen Katheter ist das distale Ende zu einem Bogen biegbar. Dieser Bogen bildet dann mit der ebenfalls gebogenen zuführenden Vene eine Ebene. Betrachtet man nun den Querschnitt der Vene so beschreibt die äußere Seite des Bogens, also die zur anderen, rechten Kammer des Herzens gerichteten Seite eine 12:00 Uhr Position und die auf der inneren Seite des Bogens liegende Stelle eine 6:00 Uhr Position.

Die Bauform des Applikationsbestecks ist schlank und flexibel, der distale Abschnitt in seiner Biegung steuerbar. Die Größe des Katheterschaftes beträgt maximal 14 French, typischerweise zwischen 8 und 12 French. Der distale Teil des Applikationsbestecks trägt den Stent mit der darin befestigten Klappe, die nur wenige Millimeter mehr Umfang hat als der Katheterschaft, typischerweise 2 mm, auch zwischen 0,5 und 4 **mm.** Dabei ist die Biegung des Katheterschaftes bzw. des distalen Teils des Applikationsbestecks steuerbar, typischerweise in einem Bereich von -20 bis etwa 210 Grad. Das Applikationsbesteck erlaubt es, die Klappe in zuvor definierter Ausrichtung zu laden, an den Bestimmungsort zu bringen und in definierter anatomischer Position freizusetzen.

Der Stent der erfindungsgemäßen Prothese weist in einer bevorzugten Ausführungsform eine Positionierhilfe auf, die Applikation bis nur an einer hierfür vorgesehen Stelle geladen werden kann. Nur an dieser Stelle passt die Positionshilfe in das Besteck, wie ein Schlüssel ins Schlüsselloch. Es hat sich als zweckmäßig erwiesen die Positionierhilfe in der 6 - 8 Uhr, vorzugsweise in der 6,5 - 7,5 Uhr Position gemäß der zuvor beschriebenen Lage anzuordnen. Außerdem hat es sich auch als zweckmäßig erwiesen am Stent röntgenopaque und/oder im Ultraschall sichtbare Markierungen anzuordnen, welche dem anwendenden Arzt eine Kontrolle der Lage des Stents und damit der Klappensegel ermöglichen.

Die Erfindung soll an den folgenden Figuren beispielhaft erläutert werden.

Es zeigen:
Fig. 1 zeigt eine stentartige Herzklappenhalterung bzw. Herzklappenhalteelement 1.0. Die stentartige Halterung bzw. Gitter enthält ein Fixierelement 1.1 zum Fixieren des stentartigen Elements im Herzen. Ein solches Fixierelement 1.1 ist vorzugsweise in Form einer Öse ausgebildet. Die Öse selbst ist von der Mittelachse des stentartigen, gitterförmigen Elements (Fließrichtung des Blutes) nach außen gebogen, sodass es sich im Herzmuskel verankert. Darüber hinaus weist es um seine zylinderartige Mantelfläche eine Einschnürung 1.2 auf. Entlang dieser Einschnürung 1.2 hat der Stent bzw. das stentartige Gitter einen geringeren Umfang aufweist als an seinen beiden distalen Enden. An seinem anderen, den fixierten Elementen gegenüberliegenden distalen Ende weist das Gitter der Halterung Halteösen 1.3 zum Befestigen der Halterung an einer hierfür vorgesehenen Stelle im Applikationsbesteck bzw. Katheter auf, wobei mindestens ein Halteelement mit einem Marker 1.4 versehen ist, um die Lage einer im Herzen geöffneten Klappenhalterung bzw. Stents überprüfen zu können. Derartige Marker sind vorzugsweise röntgenopak. Prinzipiell ist es auch möglich, derartige Marker durch Materialien bereitzustellen, die im Ultraschall besonders gut zu sehen sind.

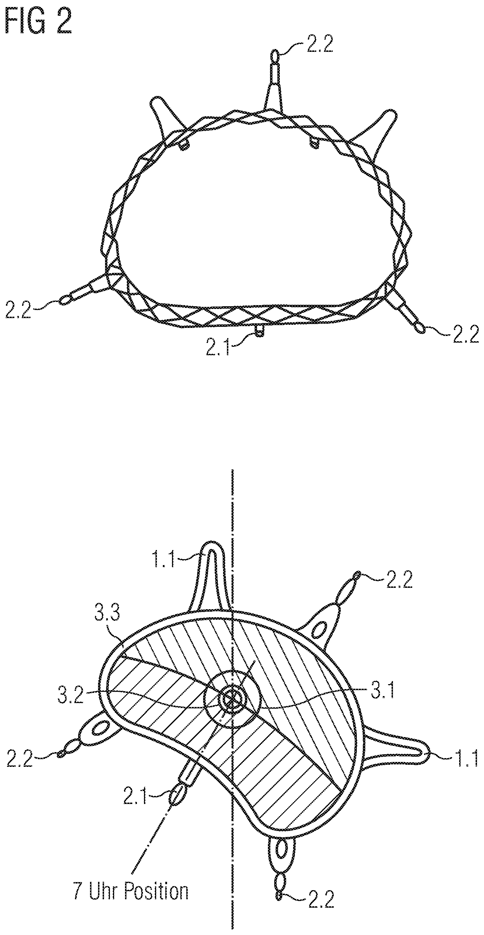

Fig. 2 zeigt eine Frontansicht bzw. Querschnitt auf das stentartige Haltegitter bzw. Element von Figur 1. Wie daraus ersichtlich ist, ist ein derartiger Stent vorzugsweise nicht vollständig rund, sondern ist zumindest an einer Seite abgeflacht. Auf diese Weise passt er sich der natürlichen Form an der Stelle der Durchtrittsöffnung am Herzen zwischen Vor- und Hauptkammer an.

Fig. 1 und 2 zeigen den grundsätzlichen Aufbau des Stentgerüstes der Klappe. Die Form ist an die natürliche Form der natürlichen Mitralklappe angepasst. Es finden sich vorzugsweise auf Vorhofseite eine oder mehrere Lageösen (1.4) zum Beladen der Prothese, die nur eine zuvor definierte Lage im Haltesystem des Applikationsbesteckes/Kathetersystems (Fig. 3, Fig. 4) erlauben und bis kurz vor der Freisetzung dazu dienen, die Herzklappe wieder in das Applikationsbesteck zurückzuziehen, falls dies zur Bergung oder erneuten Positionierung notwendig sein sollte oder noch keine endgültige Freisetzung gewünscht ist. Die Lageöse wird an einer Stelle des Katheters formschlüssig festgehalten.

Weiterhin findet sich auf Höhe des nativen Mitralrings eine Einschnürung (1.2) der stentförmigen Herzklappenhalterung, die eine definierte Höhe ermöglicht und gleichzeitig der Stabilität in der anatomisch korrekten Position dient.

Ventrikelseitig finden sich runde Ausformungen bzw. Fixierelemente des Stents (1.1), die ebenfalls der korrekten anatomischen Höhe und Stabilität des Systems dienen.

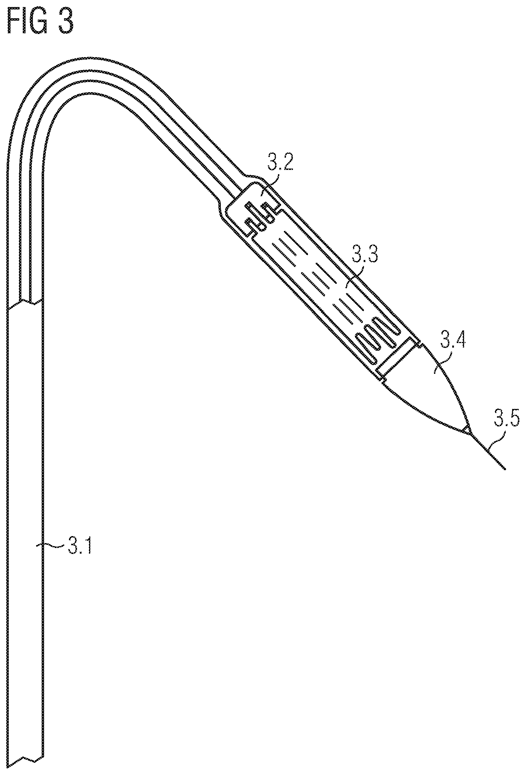

Fig. 3 zeigt ein Kathetersystem zum Legen der erfindungsgemäßen Herzklappe mit einem geladenen Stent/Implantat 3.3 der vom Stenthalter 3.2 im Kathetersystem 3.1 gehalten wird. Verschlossen ist das Kathetersystem vorne durch eine flexible Spitze 3.4, geführt durch einen Führungsdraht 3.5.

Das Kathetersystem 3.1 und der Halter für das stentartige Haltegerüst 3.2 sind daher vorzugsweise derart aufgebaut, dass bei Draufsicht auf die Katheterspitze bei der "7 Uhr" Position eine Aussparung zur Aufnahme des Zentrierelementes bzw. der Zentrieröse 2.1 angeordnet ist, sodass das stentartige Halteelement nur in dieser Position im Katheter geladen werden kann. Am Stent/Implantat 3.3 befindet sich das Zentrierelement 2.1. Das Zentrierelement hat dabei zwei insbesondere Funktionen, nämlich erstens den Stent ausschließlich und nur in der richtigen Lage zu laden, und zweitens die stentartige Halterung bzw. das ganze Implantat zu fixieren. Die Halteösen 1.3 und 1.4 am stentartigen Haltegerüst 1.0 haben ebenfalls die Aufgabe, die Prothese am Halter 3.2 zu fixieren, damit der Stent gleichmäßig und ohne "zu springen" entlassen bzw. positioniert werden kann. Die Halteösen 1.3 sind zusätzlich mit Markern ausgestattet, die Marker zeigen die Position und Lage des Stents nach der Positionierung, außerdem die Kontrolle des geöffneten Stents/Implantat.

Die definierte anatomische Position entspricht weitestgehend der natürlichen Form der Mitralklappe. Bei der Freisetzung der Klappe richtet sich diese nochmals in der Größenordnung zwischen -30 und +30 Grad, typischerweise zwischen 15 und 25 Grad und insbesonders bis zu 20 +/- 3 Grad Drehung an der natürlichen Anatomie aus.

Die Ausrichtung des Kathetersystems in Bezug auf die native Mitralklappe definiert sich durch die Ebene, die durch das gebogene Kathetersystem senkrecht vom Vorhofseptum definiert wird und die Klappenebene ebenfalls senkrecht schneidet. Diese virtuelle Ebene schneidet eine auf der Mitralklappe gedachte liegende Uhr bei 12 und 6 Uhr. Die Position des Stents mit der Klappe ist im Katheter um 30 Grad nach rechts gedreht, sodass der Positionsmarker in die 7-Uhr Position zeigt (Fig. 2).

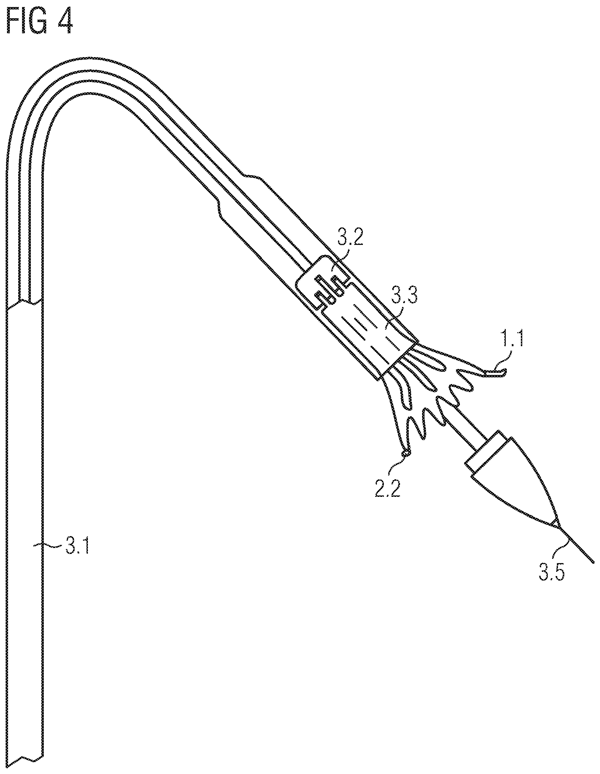

Fig. 4 zeigt einen teilentlassenen Stent/Implantat 3.3 bei dem die Positionierösen 2.2 die Position bzw. Lage des Stents/Implantat bei der Durchleuchtung zeigen. Durch die Positionierösen wird die richtige Lage des Stents/Implantats angezeigt, außerdem haben die Positionierösen auch die zusätzliche Funktion einer Halteöse, d.h. die Halteöse gilt als Anschlag an der alten Klappe. Die Fixierösen 1.1 fixieren den Stent/Implantat zusätzlich in der abgelegten Position.

Der horizontale Querschnitt der erfindungsgemäßen Klappenhalterung 1.0 hat vorzugsweise die Form einer Bohne oder Niere. Durch diese Formgebung und durch die geladene "7 Uhr" Position im Kathetersystem 3.1, hat die Klappenhalterung (Stent/Implantat) 1.0 beim Entlassen aus dem Kathetersystem 3.1 schon die vorgegebene optimale Richtung bezüglich Position und Lage im Herzen.

Fig. 2 zeigt die Draufsicht des Stents/Implantat 3.3, hier ist die Zentrieröse 2.1 sehr gut in der "7 Uhr" sichtbar.

Durch die erfindungsgemäßen Maßnahmen der vordefinierten Beladung im Applikationsbesteck, dessen steuerbares und definiertes Biegungsverhalten und die Ausrichtung der Klappenebene dazu sowie die selbstausrichtenden Eigenschaften der Klappe (Rotation und Höhe) ist die Implantation mit üblichen Bildgebungsverfahren und üblicher Expertise der Untersucher in der Bildgebung möglich. Für die Bildgebung ausreichend ist die konventionelle Röntgendurchleuchtung mit Durchlaufangiographie oder rechtsventrikulärer Angiographie oder Laevokardiographie und transthorakaler sowie transösophagealer Echokardiographie in zweidimensionaler oder mehrdimensionaler Auflösung. Alternativ kann auch intrakardialer Ultraschall (ICE) zur Anwendung kommen.

Es hat sich auch als zweckmäßig erwiesen, zusätzlich unterschiedliche Positionierhilfen anzubringen. Dabei hat es sich ebenfalls als vorteilhaft erwiesen, diese Positionierhilfen unterschiedlich auszubilden, so dass während der Implantation der behandelnde Arzt jederzeit erkennt, wie er die stentartige Halterung sicher und leicht in die richtige, zu implantierende Lage drehen kann.

Derartige Positionierhilfen sind beispielsweise röntgenopak ausgebildet. Prinzipiell ist es auch möglich, diese mit anderen zum Beispiel in einen Ultraschall gut sichtbaren Mittel zu verwenden.

Fig. 5 zeigt ein stentartiges Halteelement in Form einer Bohne, Niere oder Banane bei Draufsicht in Fließrichtung des Blutes sowie bei einer Seitenansicht.

Diese Seitenansicht zeigt ein stentartiges Halteelement, das zwei gegenläufige Trichter aufzeigt, das an der schmalsten Stelle eine Einschnürung 1.2 aufweist. Diese Einschnürung dient zur Zentrierung und Lage des abgesetzten Stents in der Klappenebene. Außerdem hat die Einschnürung auch die Funktion der Arretierung des Stents/Implantates 3.3 und ist dadurch nach oben und unten fixiert.

Fig. 6 a zeigt einen Schnitt durch die erfindungsgemäße Mitralklappenprothese 1.0 mit Segeln 6.1 und diese haltenden Sehnen bzw. Segelfäden 6.2 und der Einschnürung 1.2.

Die Adaptionslinie zwischen A1/P1 zum Ort der natürlichen Kommissur zwischen A3/P3 ist der Fig. 6 c zu entnehmen.

Fig. 7 zeigt segmentale Anatomie einer natürlichen Mitralklappe mit Lage der anterioren A1 - A3 Kommissur und der posterioren P1 P3 Kommissur.

Figur 8 zeigt eine besonders bevorzugte Ausführungsform des Steges der erfindungsgemäßen Herklappenprothese. Wie daraus entnehmbar ist, wird der Querschnitt durch zwei an ihrer senkrechten Seite gegeneinanderliegenden Formen gebildet jeweils ein großes D darstellen. Dabei weißt der größere Teil des Querschnitts mit der großen D-Form einen maximalen Abstand von der gedachten geraden D-Linie d₁ auf. Der Bogen des (kleineren linken) D weißt einen maximalen Abstand d₂ von der gedachten geraden Linie auf. Die gesamte Breite des Stents (von maximaler D-Wölbung linke Seite zur maximaler D-Wölbung rechte Seite), beträgt daher d₁ + d₂. Die maximale Länge des D (entspricht der gedachten senkrechten D-Linie des Buchstabens D) wird durch die Länger a dargestellt. In Figur 8b zeigt die erfindungsgemäß bevorzugte Form des Stentquerschnittes des der Herzklappenprothese. Dabei weist die in Fig. 8 dargestellte Form insbesonders in ihrer linken Seite eine geringere Stegbreite auf.

### Bezugszeichenliste

- 1.0: Herzklappenhalterung
- 1.1: Fixierelement
- 1.2: Einschnürung
- 1.3: Halteöse
- 1.4: Lageöse
- 2.1: Zentrieröse
- 2.2: Positionierösen
- 3.1: Kathetersystem
- 3.2: Fixierhalter
- 3.3: Implantat (Stentgitter und Segel)
- 3.4: Katheterspitze
- 3.5: Führungsdraht
- 6.1: Mitralklappensegel
- 6.2: Mitralklappenfaden

## Patentansprüche

1. Herzklappenprothese zur minimalinvasiven intravenösen Implantation, umfassend ein biologisch verträgliches, flexibles Material mit mindestens einem beweglichen Klappenflügel zum Öffnen und Schließen einer Durchtrittsöffnung zwischen Vorkammer und Hauptkammer des Herzens die an ein Halteelement aus einem stentartigen zylindrisch ausgebildeten Gitter befestigt ist, das aus einem radial aufweitbaren Material besteht, wobei das stentartige Gitter in der Aufsicht auf den Klappenapparat einen Querschnitt in Form ein doppeltes an ihrer Rückseite sich berührendes D's aufweist, **dadurch gekennzeichnet, dass** der durch ein Doppel D gebildete Querschnitt derart ausgestaltet ist, dass eines der D seitenverkehrt an der geraden Rückenlinie des anderen D zu liegen kommt mit seiner gedachten geraden Rückenlinie, wobei eines der beiden D eine größere Krümmung aufweist als das andere D.

2. Herzklappenprothese nach Anspruch 1, **dadurch gekennzeichnet, dass** das stentartige Gitter auf der Seite des D mit geringerer Wölbung, Stege mit einer geringeren Stegbreite aufweist als die Stege auf der Stentseite mit der größeren Wölbung.

3. Herzklappenprothese nach Anspruch 1, **dadurch gekennzeichnet, dass** das Verhältnis des maximalen Abstandes der größeren Krümmung von der gedachten geraden Rückenlinie des D zum entsprechenden maximalen Abstand, des anderen D mit kleinerer Krümmung 20:1 mm bis 2:1 mm beträgt.

4. Herzklappenprothese nach Anspruch 1, **dadurch gekennzeichnet, dass** das stentartige Gitter umlaufende atraumatische Halteelemente zur Fixierung an der geschädigten Mitralklappe aufweist.

5. Herzklappenprothese nach einem der vorhergehenden Ansprüche, **dadurch gekennzeichnet, dass** das stentartige Gitter eine Positionierhilfe aufweist.

6. Herzklappenprothese nach einem der vorhergehenden Ansprüche, **dadurch gekennzeichnet, dass** das stentartige Gitter eine Höhe von 20-50 mm aufweist.

7. Herzklappenprothese nach einem der vorhergehenden Ansprüche, **dadurch gekennzeichnet, dass** das verträgliche Material ein biologisches Gewebe ist, ausgewählt aus Autograft, Human-Graft, Xenograft oder aus Collagenzüchtungen.

8. Herzklappenprothese nach einem der vorhergehenden Ansprüche, **dadurch gekennzeichnet, dass** die Länge des Gitters so gewählt ist, dass es mindestens 2 mm in den Vorhof hineinragt.

9. Herzklappenprothese nach einem der vorhergehenden Ansprüche, **dadurch gekennzeichnet, dass** das stentartige Gittermaterial Nitinol ist.

10. Herzklappenprothese nach einem der vorhergehenden Ansprüche, **dadurch gekennzeichnet, dass** das stentartige Gitter nach außen gerichtete Halteelemente aufweist.

11. Herzklappenprothese nach einem der vorhergehenden Ansprüche, **dadurch gekennzeichnet, dass** das stentartige Gitter einen Querschnitt aufweist, der vom Vorhof zur Durchtrittsöffnung abnimmt und in Fließrichtung nach Durchtrittsöffnung wieder zunimmt.

12. Herzklappenprothese nach einem der vorhergehenden Ansprüche, **dadurch gekennzeichnet, dass** die Prothese zwei mitraförmige bikuspide Klappenflügel aufweist.

13. Kit of Parts, umfassend eine Herzklappenprothese nach einem der Ansprüche 1 - 12 sowie eine Implantiervorrichtung zum Implantieren von solchen Herzklappenprothesen, **dadurch gekennzeichnet, dass** sie einen steuerbaren Katheter umfasst und ein Element ausschließlich zur Aufnahme als Positionierhilfe aufweist.

14. Kit of Parts nach Anspruch 13, **dadurch gekennzeichnet, dass** die Positionierhilfe in der "7 Uhr"-Position angeordnet ist.

## Claims

1. A heart valve prosthesis for minimally invasive intravenous implantation, comprising a biocompatible flexible material having at least one movable valve leaf for opening and closing a passage opening between the atrium and the ventricle of the heart, which opening is fastened to a retaining member of a stent-like cylindrical mesh made of a radially expandable material, wherein the stent-like mesh has a cross-section in the form of a double D contacting at its rear side when viewed from the valve apparatus, **characterized in that** the cross-section formed by a double D is such that one of the Ds comes to lie laterally opposite the straight line of the back of the other D with its imaginary straight back line, wherein one of the two Ds has a greater curvature than the other D.

2. The heart valve prosthesis of claim 1, **characterized in that** the stent-like mesh on the side of the D with the smaller curvature has webs with a smaller web width than the webs on the stent side with the greater curvature.

3. The heart valve prosthesis of claim 1, **characterized in that** the ratio of the maximum distance of the greater curvature from the imaginary straight back line of the D to the corresponding maximum distance of the other D with the smaller curvature is 20:1 mm to 2:1 mm.

4. The heart valve prosthesis of claim 1, **characterized in that** the stent-like mesh comprises circumferential atraumatic retaining members for fixing to the damaged mitral valve.

5. The heart valve prosthesis of any one of the preceding claims, **characterized in that** the stent-like mesh has a positioning aid.

6. The heart valve prosthesis of any one of the preceding claims, **characterized in that** the stent-like mesh has a height of 20-50 mm.

7. The heart valve prosthesis of any one of the preceding claims, **characterized in that** the compatible material is a biological tissue selected from autograft, human graft, xenograft, or collagen cultures.

8. The heart valve prosthesis of any one of the preceding claims, **characterized in that** the length of the mesh is selected such that it projects at least 2 mm into the atrium.

9. The heart valve prosthesis of any one of the preceding claims, **characterized in that** the stent-like mesh material is nitinol.

10. The heart valve prosthesis of any one of the preceding claims, **characterized in that** the stent-like mesh has outwardly directed retaining members.

11. The heart valve prosthesis of any one of the preceding claims, **characterized in that** the stent-like mesh has a cross-section which decreases from the atrium to the passage opening and increases again in the flow direction after the passage opening.

12. The heart valve prosthesis of any one of the preceding claims, **characterized in that** the prosthesis comprises two mitral-shaped bicuspid flaps.

13. A kit of parts comprising a prosthetic heart valve of any one of claims 1 - 12 and an implantation device for implanting such heart valve prostheses, **characterized in that** the device comprises a controllable catheter and has an element exclusively for accommodation as a positioning aid.

14. The kit of parts of claim 13, **characterized in that** the positioning aid is arranged in the "7 o'clock" position.

## Revendications

1. Prothèse valvulaire cardiaque pour implantation intraveineuse minimalement invasive, comprenant un matériau flexible biologiquement compatible ayant au moins une ailette valvulaire mobile pour ouvrir et fermer un orifice de passage entre l'oreillette et la chambre principale du cœur fixé à un élément de retenue d'une grille cylindrique en forme de stent constituée d'un matériau dilatable radialement, dans lequel la grille en forme de stent présente, en vue de l'appareil valvulaire, une section transversale en forme de double D en contact avec sa face arrière, **caractérisé en ce que** la section transversale en forme de double D est conçue de sorte que l'un des D vient se placer de manière latéralement inversée sur la ligne droite arrière de l'autre D avec sa ligne droite arrière imaginaire, dans lequel l'un des deux D présente une courbure plus grande que l'autre D.

2. Prothèse valvulaire cardiaque selon la revendication 1, **caractérisée en ce que** la grille en forme de stent présente, du côté du D avec une courbure plus faible, des nervures avec une largeur de nervure plus faible que les nervures du côté du stent avec la courbure plus grande.

3. Prothèse valvulaire cardiaque selon la revendication 1, **caractérisée en ce que** le rapport entre la distance maximale de la courbure la plus grande entre la ligne droite arrière imaginaire du D et la distance maximale correspondante de l'autre D de courbure la plus petite est de 20:1 mm à 2:1 mm.

4. Prothèse valvulaire cardiaque selon la revendication 1, **caractérisée en ce que** la grille en forme de stent présente des éléments de retenue non traumatiques périphériques pour la fixation sur la valve mitrale endommagée.

5. Prothèse valvulaire cardiaque selon l'une quelconque des revendications précédentes, **caractérisée en ce que** la grille en forme de stent présente une aide au positionnement.

6. Prothèse valvulaire cardiaque selon l'une quelconque des revendications précédentes, **caractérisée en ce que** la grille en forme de stent présente une hauteur de 20 à 50 **mm.**

7. Prothèse valvulaire cardiaque selon l'une quelconque des revendications précédentes, **caractérisée en ce que** le matériau compatible est un tissu biologique choisi parmi une autogreffe, une greffe humaine, une xénogreffe ou des cultures de collagène.

8. Prothèse valvulaire cardiaque selon l'une quelconque des revendications précédentes, **caractérisée en ce que** la longueur de la grille est choisie de sorte qu'elle fait saillie d'au moins 2 mm dans l'oreillette.

9. Prothèse valvulaire cardiaque selon l'une quelconque des revendications précédentes, **caractérisée en ce que** le matériau de la grille en forme de stent est du Nitinol.

10. Prothèse valvulaire cardiaque selon l'une quelconque des revendications précédentes, **caractérisée en ce que** la grille en forme de stent présente des éléments de retenue orientés vers l'extérieur.

11. Prothèse valvulaire cardiaque selon l'une quelconque des revendications précédentes, **caractérisée en ce que** la grille en forme de stent présente une section transversale qui diminue de l'oreillette vers l'orifice de passage et qui augmente à nouveau dans le sens de l'écoulement vers l'orifice de passage.

12. Prothèse valvulaire cardiaque selon l'une quelconque des revendications précédentes, **caractérisée en ce que** la prothèse présente deux ailettes valvulaires bicuspides en forme de mitre.

13. Kit de pièces comprenant une prothèse valvulaire cardiaque selon l'une quelconque des revendications 1 à 12 ainsi qu'un dispositif d'implantation pour implanter de telles prothèses de valve cardiaque, **caractérisé en ce qu'**il présente un cathéter pouvant être commandé et un élément destiné exclusivement à être reçu comme aide au positionnement.

14. Kit de pièces selon la revendication 13, **caractérisé en ce que** l'aide au positionnement est disposée dans la position « 7 h ».
